# EUROPEAN PATENT SPECIFICATION

(11) **EP 0 453 112 B1**
(45) Date of publication and mention of the grant of the patent: **08.01.1997**
(21) Application number: 91302739.7
(22) Date of filing: 28.03.1991
(51) Int. Cl.: C08K 5/00, C08K 5/59

(54) **Microbicides immobilized in water-soluble thermoplastic polymeric resins and aqueous dispersions of microbicides prepared therefrom**
In Wasser lösliche, thermoplastische, polymere Harze, immobilisierte Mikrobizide und daraus hergestellte wässrige Mikrobizid-Dispersionen
Microbicides immobilisés dans des résines polymères thermoplastiques solubles dans l'eau, et dispersions aqueuses de microbicides préparées à partir de celles-ci

(30) Priority: 02.04.1990 US 503359
(43) Date of publication of application: 23.10.1991
(73) Proprietor: MORTON INTERNATIONAL, INC., Chicago, Illinois 60606 (US)
(72) Inventor: Rei, Nuno M., Boxford, MA 01921 (US); Grant, Lawrence P., Danvers, MA 01923 (US); Hamel, Roger G., Methurn, MA 01844 (US)
(74) Representative: Bankes, Stephen Charles Digby

(56) References cited:
- EP-A- 0 259 946
- FR-A- 2 332 765
- GB-A- 2 095 558
- US-A- 3 299 566
- US-A- 4 155 742
- US-A- 4 888 175

## Description

The present invention is directed to solid microbicide concentrates in which microbicides are immobilized in water-soluble resins. Such concentrates are particularly useful as additives to solid resins as a means of imparting biocidal activity thereto.

It is known to protect various thermoplastic resin compositions against fungal or bacterial attack by incorporating a microbicide therein to prevent the deterioration of articles formed from the resin compositions. Microbicides inhibit growth of bacteria or fungi by acting upon the cell wall or upon cell proteins, e.g., by attacking disulfide bonds. In order for the microbicide to be effective in the resin composition, it is necessary that it be compatible with the components of the resin composition and be uniformly dispersible in the resin composition. The microbicide must be carried by the resin composition in a manner that it remains biologically active against microorganisms, and, in particular, must be available at the surfaces, including internal pore surfaces. Incorporation of microbicides in resin compositions is generally effective only in compositions in which the microbicide is able to slowly migrate to the surfaces. In some cases, the microbicide migrates slowly through amorphous regions of the polymer. In other cases, biocide migration is facilitated by plasticizers which are included along with the polymeric resins in end-use resin compositions. As the microbicide at the surfaces is used up through action against microorganisms, additional microbicide migrates to the surfaces. Although a microbicide may be a highly toxic chemical, its low concentration in the end-use product and its retention by the resin composition ensures that the microbicide in the end-use product poses no hazard to humans or animals.

Microbicides must be available in a form that is readily dispersible into the formulation mix from which the end-use resin composition is fabricated. The powdered or crystalline forms in which many useful microbicides are commercially available are readily dispersible; however, at the site of mixing, powdered or crystalline microbicides pose a substantial environmental and health hazard if powder or crystal fines are dispersed into the atmosphere. Furthermore, powder, or powdered fines, if dispersed into the atmosphere, represent a potential explosive hazard.

Recognizing the toxicity problem of microbicides in powder or crystalline form, U.S. Patent No. Re. 29,409 teaches dissolving microbicides in liquid solvents which may be added to the formulation mixture from which the end-use resin compositions are fabricated. Although liquid dispersions may be safely used at the site of preparing end-use resin compositions, careless use or disposal of the liquids may still pose environmental and health hazards.

US-A-4,086,297 describes solid thermoplastic microbicide resin concentrates containing immobilized microbicides. These solid microbicide resin concentrates contain relatively high concentrations of microbicides and may be added to the formulation mixtures from which the end-use resin compositions are prepared in an amount sufficient to provide the desired end-use microbicide concentrations. The solid microbicide resin concentrates, which are typically provided in the form of small pellets, can be handled freely, posing substantially no health or environmental threat. Such pellets are even safe for direct skin contact. Although microbicides are sufficiently immobilized and inactive in the solid microbicide resin concentrates, in softer end-use resin compositions, the low concentration microbicides at the surface have biological activity, and gradual and continuous migration to surfaces ensures continuous biological activity. Where practical, a solid microbicide resin concentrate represents a preferred manner of providing a microbicide to producers of end-use thermoplastic products.

US-A-4,789,692 discloses blends of polymers and also copolymers and terpolymers that are particularly suitable for carrying concentrated levels of microbicides into particular thermoplastic resins.

In order that inclusion of a microbicide impart biocidal activity to an end-use product, the microbicide must be available at the surface to act against microbial growth. In a flexible end-use composition, which comprises a thermoplastic resin and a plasticizer, the plasticizer commonly provides the transport mechanism for continual replenishment of incorporated microbicide to the surface of the end-use article. A typical product of this nature is a polyvinyl chloride (PVC) shower curtain which contains substantial amounts of plasticizer and sufficient microbicide to protect the shower curtain from microbial attack for an extended period of time.

On the other hand, rigid polymeric materials may be afforded substantially no protection by the inclusion of microbicides because the incorporated microbicide does not migrate to the surface where it is available to act against microbial organisms. An example of this is rigid PVC, such as that for siding of houses. Unlike flexible, plasticized PVC compositions, rigid, non-plasticized PVC is not particularly subject to degradation by microbial attack. Nevertheless, microbial growth on the surface of such rigid polymeric material is undesirable, particularly from an aesthetic standpoint.

According to one aspect of the invention there is provided a method of dispersing a solid microbiocide in a rigid thermoplastic resin composition wherein the microbiocide is dissolved in a carrier to form a concentrate and the concentrate is dispersed in said resin composition, characterised in that said concentrate is a solid solution, or suspension of microcrystals of the microbiocide in a thermoplastic polymeric resin which is soluble in water to at least 1g per 100ml of water at 25°C.

According to a further aspect of the invention there is provided a polymeric resin composition obtainable by the process defined above comprising, a primary thermoplastic resin or mixture of such resins, at least 0.03 wt.% of a microbiocide to protect said composition from surface microbial growth and a thermoplastic polymeric resin which is soluble in water to at least 1g per 100ml of water at 25°C, wherein the weight ratio of the total resin composition to the microbiocide is at least 20 times the weight ratio of said water-soluble resin to the microbiocide.

One aspect of the present invention is directed to concentrates of microbiocides in water-soluble thermoplastic resins, which concentrates are used to carry microbiocides into end-use resin compositions. It is found that water-soluble thermoplastic resins promote mobility of microbiocides in end-use resin compositions, promoting migration of the microbiocides to surfaces (including pore surfaces) of the end-use resin compositions. It is particularly difficult to protect surfaces of rigid or glassy thermoplastic resin compositions from surface microbial growth by incorporation of microbicides, because incorporated microbicides tend to be immobilized in such compositions. When the microbicide at the surface of such a composition is depleted, it is not replenished by migration of microbicide incorporated internally, or at least not at a sufficient rate. Thus, by use of the water-soluble thermoplastic resins as microbicide carriers in accordance with the invention, thermoplastic resin compositions, which heretofore could not be adequately protected by incorporating microbicides, may now be protected. Concentrates according to the invention have been shown to impart biological activity to rigid compositions of polyethylene terephthalate (PET) acrylonitrile-butadiene-styrene polymer (ABS), polyvinyl chloride (PVC), polyvinylidene chloride (PVDC), polycarbonate and polystyrene.

Rigid or glassy thermoplastic resin compositions are generally those having a glass transition temperature above room temperature, e.g., above about 25°C. However, whether or not microbicide will be migratory or non-migratory in an end-use resin composition or whether, if migratory, at any appreciable rate, will depend upon a variety of factors, including the chemical composition of the resin or resins and the additives to the end-use resin composition, such as plasticizers, flow-control agents, fillers, etc. In any case, it is believed that the water-soluble thermoplastic carrier resins used in accordance with the present invention generally promote microbicide migration in end-use thermoplastic resin compositions. Thus, while a particularly important application of the concentrates is to enable solid or glassy thermoplastic resins to be protected by incorporated microbicides, the concentrates have broad applications to thermoplastic resin compositions. For example, a non-rigid, thermoplastic, end-use resin composition for which a short life-span is anticipated, e.g., a garbage bag, may require less total amount of microbicide if the migration rate of the microbicide is enhanced.

By aqueous-soluble thermoplastic resin is meant a thermoplastic resin which is soluble to at least 1 g. per 100 ml, and preferably at least 5 g. per 100 ml of water at 25°C.

In order to be useful as carriers of microbicides, it is necessary that the resins be thermoplastic, and in this respect, the carrier resins must each melt at a temperature below its decomposition temperature. Concentrates according to the present invention are prepared by melt-blending the carrier resins and the microbicides. Furthermore, to be useful as a carrier in association with any end-use thermoplastic resin composition, the carrier resin should be stable to degradation at the processing temperature of the end-use thermoplastic resin composition.

In selecting a water-soluble resin as a carrier for a particular microbicide into an end-use resin composition, an important criteria is that the resin be capable of solubilizing a high concentration of the particular microbicide. In most cases, the addition of the water-soluble carrier resin is considered to be an undesirable addition to the rigid thermoplastic resin or at least imparts no advantageous properties to the end-use thermoplastic resin composition other than facilitating migration of the microbicide. To minimize levels of water-soluble resin added to the end-use resin composition, it is therefore generally considered that the higher the concentration of microbicide in the water-soluble carrier resin, the better. Of course, sufficient water-soluble resin must be added to the end-use resin composition to facilitate migration of the microbicide to the surface of the end-use resin composition during the life of the end-use product; however, solubility of the microbicide in the water soluble resin is generally the limiting factor.

It is usually necessary only that the microbicide be soluble in the molten water-soluble carrier resin. Depending upon the particular resin, the particular microbicide and the concentration, the concentrate in solid form may have the microbicide still fully dissolved in the solidified resin. However, it is permissable for many applications that the microbicide recrystallize to some extent upon solidification of the water-soluble carrier resin. In such case, the crystals of microbicide which form will be of very small size and will distribute evenly throughout an end-use resin composition or will be of a size suitable for other applications of the present invention hereinafter discussed.

A concentrate in accordance with the invention should contain at least 20 times the concentration of microbicide that is to be present in the end-use thermoplastic resin composition, preferably at least 100 times the end-use concentration. Depending upon the microbicide, the concentrate may contain up to 1000 times the end-use concentration. The concentrate is added to the end-use resin in an amount sufficient to provide-the desired concentration of microbicide to the end-use product. Thus, for example, if the concentrate contains 100 times the end-use concentration of microbicide, it will be added to the thermoplastic resin of the end-use composition at a weight ratio of 1:99. The end-use concentration is that required in the end-use resin composition to prevent microbial growth thereon. The end-use concentration will vary widely, depending upon the particular microbicide used; however, selection of an appropriate end-use concentration is believed to be within the skill of one with ordinary skill in the art, particularly with reference to published activity levels of various microbicides.

End-use concentrations of several commercially-available microbicides in various types of resins are given in the table below:

| Applications/Use Levels | | | | | | |
|---|---|---|---|---|---|---|
| Active Ingredients | Vinyl | Olefins | TPU | PU Foam | EVA | Nylon |
| OBPA | 0.03 to 0.05% | 0.05 | .05 | .05 | 0.05 | 0.05 |
| T-129 | .25% | UNK | 0.25 | | -- | NA |
| Irgasan (Ciba Geiby) | NA | 0.1-0.5% | UNK | UNK | 0.05 | 0.05% |
| Kathon-893 (M (Rohm & Haas) | 2-4 phr | 0.05% | UNK | UNK | 0.5% | NA |
| Vancide PA (R.T. Vanderbilt) | 0.5% | NA | UNK | UNK | 0.5% | NA |
| Daconil 2787 (SDS Biotech) | 0.5 to 1% | UNK | UNK | UNK | UNK | |
| Preventol (Bayer) | 0.25 to 0.5% | UNK | UNK | UNK | UNK | UNK |
| TBZ (Merk/Carbonl) | >0.25% | NA | UNK | UNK | NA | NA |
| Zinc omadine (Olin) | 0.2% | UNK | UNK | UNK | UNK | UNK |

The water-soluble thermoplastic resin is further selected according to the primary thermoplastic resin so that the water-soluble resin has minimal negative effects on the properties of the primary thermoplastic resin composition. In this regard, it may be advantageous that the water-soluble thermoplastic resin be chemically similar to the primary thermoplastic resin to which it is to be added.

Currently preferred water-soluble resin carriers are resins based primarily upon polyvinyl alcohol (PVA). Polyvinyl alcohols are hydrolysis products of polyvinyl acetate or another polyvinyl ester. Polyvinyl alcohols, without modification, tend to be non-thermoplastic, generally having decomposition temperatures below their melting points. However, polyvinyl alcohol-based resin compositions may be either externally or internally plasticized so as to exhibit thermoplastic properties. It is known for example to plasticize PVA with such plasticizers as polyethylene glycol, glycerol and neopentyl glycol and thereby give PVA compositions thermoplastic properties. Such externally plasticized PVA resins are described, for example, in U.S. Patent Nos. 3,425,979 and 4,469,837. PVA copolymers and graft polymers, such as those described in U.S. Patents Nos. 2,990,398, 1,971,662, 2,844,570, 3,033,841 and 4,369,281, also exhibit thermoplastic properties. A currently preferred internally plasticized PVA-based resin is a copolymer of vinyl alcohol and (alkyleneoxy)acrylate described in U.S. Patent No, 4,618,648. Such resins are sold under the trademark Vinex by Air Products.

Furthermore, it is desirable that the water-soluble resin be extrudable as a means of forming pellets of the concentrate. The microbicide and water-soluble resin are blended in the extruder above the softening point of the water-soluble resin, whereupon the microbicide is dissolved in the resin. Upon cooling, a solid solution (or suspension of micro crystals) of microbicide-in-resin results. Typically, the concentrates are extruded as strands and divided into pellets as they solidify.

The solid concentrates, like solid concentrates heretofore described, immobilize the toxic microbicide in a manner which is inherently safe to handle, as least greatly so relative to powdered microbicides.

The concentrates, e.g., in pellet or ground particulate form, are added to a thermoplastic resin in the conventional manner. Typically, concentrate pellets and fragmented primary thermoplastic resin are admixed along with optional additional additives in an extruder and the resin composition extruded at an appropriately elevated temperature. The end-use resin composition which results has sufficient microbicide to protect it from microbial growth and sufficient water-soluble resin to facilitate migration of the microbicide so as to provide microbicide to the surface of the end-use product over an extended lifetime. Typically, the end-use resin composition contains between about 0.1 and about 5 wt. percent of the water-soluble resin. Preferably, the end-use resin composition contains no more than about 1 wt. percent of the water-soluble resin.

Examples of the types of microbiocidal compounds which may be employed in this invention include, but are not limited to, phenoxarsines (including bisphenoxarsines), phenarsazines (including bisphenarsazines), maleimides, isoindole dicarboximides having a sulfur atom bonded to the nitrogen atom of the dicarboximide group, halogenated aryl alkanols and isothiazolinone compounds.

The microbiocidal phenoxarsine and phenarsazine compounds useful in the compositions of this invention include compounds represented by the formulas: 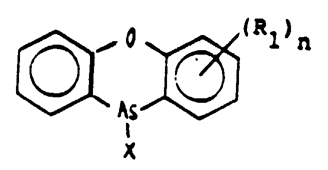 and 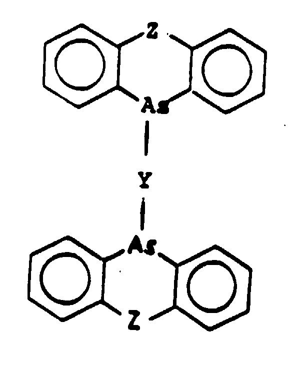 where X is halogen or thiocyanate, Y is oxygen or sulfur, Z is oxygen or nitrogen, R is halo or lower alkyl, and n is 0 to 3. Examples of these phenoxarsines and phenarsazines include, but are not limited to,
10-chlorophenoxarsine; 10-iodophenoxarsine;
10-bromophenoxarsine; 4-methyl-10-chlorphenoxarsine,
2-tert-butyl-10-chlorophenoxarsine;
1,4-dimethyl-10-chlorophenoxarsine;
2-methhyl-8,10-dichlorophenoxarsine;
1,3,10-trichlorophenoxarsine;
2,6,10-trichlorophenoxarsine;
1,2,4,10-tetrachlorophenoxarsine;
10,10'-oxybisphenoxarsine (OBPA);
10,10'-oxybisphenarsazine and 10,10'-thiobisphenarsazine.

The microbiocidal maleimide compounds useful in the compositions of this invention are exemplified by a preferred maleimide, N-(2-methylnaphtyhl) maleimide.

The microbiocidal compounds useful in the practice of this invention which are isoindole dicarboximides having a sulfur atom bonded to the nitrogen atom of the dicarboximide group are compounds which contain at least one group having the structure: 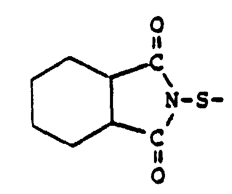 The preferred isoindole dicarboximides are the following: 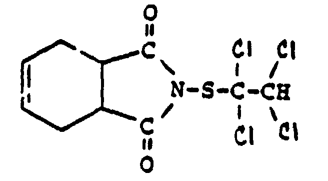 bis-[(1,1,2,2-tetrachloroethyl)thio]-4-cyclohexene-1,2-dicarboximide; 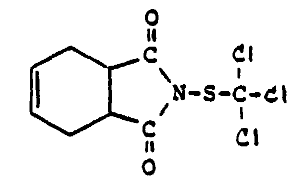 N-trichloromethylthio-4-cyclohexene-1,2-dicarboximide; and 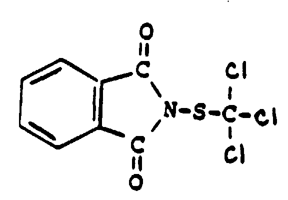 N-trichloromethylthio phthalimide.

The halogenated aryl alkanols which can be used as microbiocidal compounds in accordance with this invention are exemplified by a preferred compound, 2,4-dichlorobenzyl alcohol.

An example of a preferred isothiazolinone compound useful in the composition of this invention is 2-(n-octyl-4-isothiazolin-3-one).

The most preferred microbiocidal compounds are the bisphenoxarsines and bisphenarsazines having the formula: 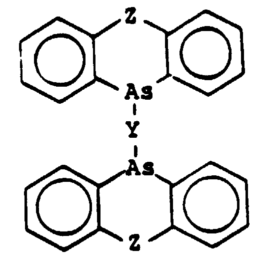 where Y is oxygen or sulfur and Z is oxygen or nitrogen. Of these bisphenoxarsines and bisphenarsazines, the most preferred are 10,10'-oxybisphenoxarsine; 10,10'-thiobisphenoxarsine; 10,10'-oxybisphenarsazine; and 10,10'-thiobisphenarsazine.

It is generally possible to incorporate at least about 1 wt %, more preferably 2 wt. %, of a bisphenoxarsine or bisphenarsazine in PVA-based, internally or externally plasticized, thermoplastic resin compositions. Using the preferred copolymer of vinyl alcohol and (alkyleneoxy)acrylate described in the above-referenced U.S. Patent No, 4,618,648, it is possible to obtain up to about 5 wt. % compositions of phenarsazines and phenoxarsines dissolved in the solidified resin.

Concentrates containing up to about 20 wt. percent of phenarsazines and phenoxarsines are obtainable with the poly(alkyleneoxy) acrylate resin which dissolves such concentrations when molten; however, upon solidification of the resin, the microbicide comes out of solution as microcrystals suspended in the solidified resin.

Concentrates of microbicides in water-soluble resins or mixtures of end-use resins may themselves be used to form end-use products that are useful for adding the microbicides to aqueous systems. For example, concentrates may be produced in film form and manufactured into water-soluble bags for use in commercial laundries. It is presently conventional in commercial laundries to add water-soluble bags containing detergents, microbicides, water softeners, etc. It may be readily appreciated that the addition of powdered microbicides to the detergent, water softener, etc., is an inherently hazardous operation. Likewise, inadvertent breakage of the bag, either at the laundry or throughout the distribution system poses a potential danger to those who might inadvertently come into contact with the hazardous mixture--probably without awareness of any such hazard. A water-soluble bag for detergents etc., in which the microbicide is dissolved in the bag material itself, greatly minimizes any potential hazard.

The invention will now be described in greater detail by way of specific examples.

### Example 1

Concentrates of 1%, 2% and 5% OBPA in Vinex 2025 (poly(alkyleneoxylacrylate)) were prepared. The Vinex 2025 was first ground using a Brinkman Mill equipped with a 4 mm screen. The Vinex granules were blended with the OBPA in a Hobart blender. The blends were then compounded using a 0.75 inch single screw extruder. The compounds processed best with extrusion temperatures of zone 1 at 165°C. zone 2 at 170°C, zone 3 at 175°C and the die at 180°C. The extruder was equipped with a 4 inch wide sheet die. The concentrate sheets were granulated using the Brabender granulator equipped with a 4 mm screen.

Four rigid polymer resins were used in this example.
1. ABS.
2. Polycarbonate (PC).
3. PET from Hoechst Celanese. This resin is used to make polyester fibers.
4. Polystyrene (PS) PS-208.

A general purpose crystalline polystyrene resin from Huntsman Chemical.

Four films were prepared from each rigid polymer film using the three Vinex concentrates. Each of these films contained 500 ppm OBPA and 1%, 2-1/2% or 5% of Vinex. An untreated control film of each resin was also prepared. Films were prepared on a single screw extruder equipped with a 4 inch sheet die. Extruder conditions are given below:

| Extrusive Condition for Rigid Films | | | | |
|---|---|---|---|---|
| | ABS | PC | PET | PS |
| Zone 1 (°C) | 180 | 270 | 270 | 190 |
| Zone 2 (°C) | 185 | 260 | 260 | 195 |
| Zone 3 (°C) | 190 | 250 | 250 | 200 |
| Die (°C) | 200 | 245 | 245 | 200 |
| | | | | |
| Screw Speed (RPM) | 125 | 125 | 125 | 125 |

The extruded films were evaluated for activity against microorganisms.

### Results:

The 1% OBPA in Vinex 2025 processed well. The 2% OBPA in Vinex 2025 processed acceptably but some slippage of the compound on the extruder screw resulted in lower output The 5% OBPA in Vinex 2025 processed poorly with slippage of the compound on the extruder screw resulting in a loud squeaking sound and erratic output.

The addition of the Vinex did not affect the processing of the ABS, PC or PS films. The PET samples containing Vinex had to be extruded at a lower temperature than the virgin resin because of a loss of hot strength of the film. They processed well at the lower temperature. The addition of Vinex to PC and PS caused them to become slightly opaque.

The biological test procedures are described in Table 1 below.

**TABLE 1**

| Biological Test on Extruded Films | | | | | |
|---|---|---|---|---|---|
| Polymer | Vinex (%) | OBPA (ppm) | Average* Zone of Inhibition(mm)/Growth in Contact Area | | Growth of Rhodotorula rubra in agar overlay |
| | | | Staphylococcus aureus | Kubsiella pneumoniae | |
| ABS | 0 | 0 | 0/GCA | 0/GCA | HG |
| ABS | 1 | 500 | 0/GCA | 0/GCA | MG |
| ABS | 2.5 | 500 | 1/NGCA | 0/GCA | LG |
| ABS | 5 | 500 | 3/NGCA | 0/GCA | NG |
| | | | | | |
| PC | 0 | 0 | 0/GCA | 0/GCA | HG |
| PC | 1 | 500 | 0/GCA | 0/GCA | HG |
| PC | 2.5 | 500 | Halo/NGCA | 0/NGCA | NG |
| PC | 5 | 500 | 4/NGCA | 2/NGCA | NG |
| | | | | | |
| PET | 0 | 0 | 0/GCA | 0/GCA | HG |
| PET | 1 | 500 | 0/GCA | 0/GCA | MG |
| PET | 2.5 | 500 | 0/GCA | 0/GCA | TG |
| PET | 5 | 500 | Halo/NGCA | 0/GCA | NG |
| | | | | | |
| PS | 0 | 0 | 0/GCA | 0/GCA | HG |
| PS | 1 | 500 | Halo/NGCA | 0/GCA | NG |
| PS | 2.5 | 500 | 5/NGCA | 2/NGCA | NG |
| PS | 5 | 500 | 8/NGCA | 3/NGCA | NG |

| | | | | | |
|---|---|---|---|---|---|
| *Average of 3 determinations HG = Heavy growth MG = Medium growth LG = Light growth TG = Trace growth NG = No growth CA = Contact area Halo = Area of inhibited growth | | | | | |

This example demonstrates that Vinex is effective in improving the migration of OBPA through rigid polymers.

### Example 2

This test was done to determine the effects of a water-soluble carrier resin on the physical properties of a rigid end-use resin. No microbicide was included because only physical properties were measured in this example.

Films of ABS (Cycolac T 4500) containing 0, 1, 5, 10 and 20% Vinex 2025 were prepared on a lab extruder. Cycolac T is the most widely used grade of general purpose ABS offered by General Electric. According to the manufacturer it is recognized as the standard of the ABS industry.

Blends containing 0, 1, 5, 10 and 20% Vinex 2025 in Cycolac T 4500 were made in the Hobart blender. Films were extruded from the blends on a 0.75 inch single screw extruder equipped with a 4 inch sheet die. They were cooled and polished on chrome rolls. The films were 20 to 25 mils thick.

The tensile strength was measured according to ASTM test method D 638 using a Type IV specimen and a crosshead speed of 5 mm/min.

The addition of the Vinex to the ABS had no effect on the processability of the films. The films containing 10% and 20% vinex had a laminar appearance when they were cut. The film with 5% Vinex showed this phenomena to a very slight extent. The Vinex is believed not to be miscible with the ABS and is believed to form a dispersed phase within the ABS continuous phase.

Table 2 contains the tensile strength measurements of the films. At up to 10% Vinex there is very little loss of physical properties. At 20% Vinex the loss in tensile is greater than 20%.

**Table 2**

| Tensile Strenght of Cycolac T with Vinex 2025 | | |
|---|---|---|
| % Vinex | Tensile Strength (psi) + S.D. | % Tensile Strenght Loss |
| 0 | 5970 + 180 | 0.0 |
| 1 | 5410 + 280 | 9.4 |
| 5 | 5530 + 140 | 7.3 |
| 10 | 5740 + 430 | 3.9 |
| 20 | 4570 + 190 | 23.5 |

## Claims (Claims for the following Contracting State(s): AT, BE, CH, DE, DK, FR, GB, GR, IT, LI, LU, NL, SE)

1. A method of dispersing a solid microbiocide in a rigid thermoplastic resin composition wherein the microbiocide is dissolved in a carrier to form a concentrate and the concentrate is dispersed in said resin composition, characterised in that said concentrate is a solid solution, or suspension of microcrystals, of the microbiocide in a thermoplastic polymeric resin which is soluble in water to at least 1g per 100ml of water at 25°C.

2. A method according to claim 1 wherein the concentrate is dispersed in a rigid primary thermoplastic resin and the microbiocide is dissolved in said water-soluble thermoplastic resin at a concentration which is at least 20 times its concentration in the final primary resin composition.

3. A method according to claim 2 wherein the microbiocide is dissolved in said water soluble resin at a concentration which is at least 100 times its concentration in the final primary resin composition.

4. A method according to any preceding claim wherein said microbiocide is a water-insoluble microbiocide.

5. A method according to any preceding claim wherein said microbiocide is a phenoxarsine or a phenarsazine.

6. A method according to claim 5 wherein said microbiocide is 10,10'-oxybisphenoxarsine.

7. A method according to any preceding claim wherein said water-soluble polymeric resin is a polyvinyl alcohol-based thermoplastic resin.

8. A method according to any preceding claim wherein said microbiocide is present in the concentrate at a concentration of at least 2 wt.%, preferably at least 5 wt.%.

9. A polymeric resin composition obtainable by a process according to any preceding claim comprising, a primary thermoplastic resin or mixture of such resins, at least 0.03 wt.% of a microbiocide to protect said composition from surface microbial growth and a thermoplastic polymeric resin which is soluble in water to at least lg per 100ml of water at 25°C.

10. A composition according to claim 9 wherein said primary thermoplastic resin(s) is a(are) halogenated vinyl resin(s).

11. A composition according to claim 10 wherein said primary thermoplastic resin is a polyvinyl chloride.

12. A composition according to any one of claims 9 to 11, wherein said water-soluble thermoplastic resin is a polyvinyl alcohol-based thermoplastic resin.

13. A composition according to any one of claims 9 to 12 wherein microbiocide is a phenarsazine or a phenoxarsine.

14. A composition according to claim 13 wherein said primary thermoplastic resin is selected from acrylonitrile-butadiene-styrene polymer, polyethylene terephthalate, polystyrene, styrene butadiene rubber, polyvinylchloride, polyvinylidene chloride and ethylene vinylacetate copolymer.

15. A composition according to any one of claims 9 to 14 wherein the microbiocide concentration is from 0.03 to 0.5 wt.%.

## Claims (Claims for the following Contracting State(s): ES)

1. A method of dispersing a solid microbiocide in a rigid thermoplastic resin composition wherein the microbiocide is dissolved in a carrier to form a concentrate and the concentrate is dispersed in said resin composition, characterised in that said concentrate is a solid solution, or suspension of microcrystals of the microbiocide in a thermoplastic polymeric resin which is soluble in water to at least 1g per 100ml of water at 25°C.

2. A method according to claim 1 wherein the concentrate is dispersed in a rigid primary thermoplastic resin and the microbiocide is dissolved in said water-soluble thermoplastic resin at a concentration which is at least 20 times its concentration in the final primary resin composition.

3. A method according to claim 2 wherein the microbiocide is dissolved in said water soluble resin at a concentration which is at least 100 times its concentration in the final primary resin composition.

4. A method according to any preceding claim wherein said microbiocide is a water-insoluble microbiocide.

5. A method according to any preceding claim wherein said microbiocide is a phenoxarsine or a phenarsazine.

6. A method according to claim 5 wherein said microbiocide is 10,10'-oxybisphenoxarsine.

7. A method according to any preceding claim wherein said water-soluble polymeric resin is a polyvinyl alcohol-based thermoplastic resin.

8. A method according to any preceding claim wherein said microbiocide is present in the concentrate at a concentration of at least 2 wt.%, preferably at least 5 wt.%.

9. A method according to any preceding claim wherein said primary thermoplastic resin is a halogenated vinyl resin.

10. A method according to any one of claims 1 to 8 wherein said primary thermoplastic resin is selected from acrylonitrile-butadiene-styrene polymer, polyethylene terephthalate, polystyrene styrene butadiene rubber, polyvinylchloride, polyvinylidene chloride and ethylene vinylacetate copolymer.

11. A method according to any preceding claim wherein the microbiocide concentration in the final resin composition is from 0.03 to 0.5 wt.%.

## Patentansprüche (Patentansprüche für folgende(n) Vertragsstaat(en): AT, BE, CH, DE, DK, FR, GB, GR, IT, LI, LU, NL, SE)

1. Verfahren zum Dispergieren eines festen Mikrobiocids in einer starren thermoplastischen Harzzusammensetzung, wobei das Mikrobiocid in einem Träger unter Bildung eines Konzentrates aufgelöst wird und das Konzentrat in der Harzzusammensetzung dispergiert wird, **dadurch gekennzeichnet**, daß das Konzentrat eine feste Lösung oder Suspension von Mikrokristallen des Mikrobiocids in einem thermoplastischen Polymerharz ist, welches in Wasser bis zu wenigstens 1 g/100 ml Wasser bei 25 °C löslich ist.

2. Verfahren nach Anspruch 1, bei dem das Konzentrat in einem starren primären thermoplastischen Harz dispergiert wird und das Mikrobiocid in diesem wasserlöslichen thermoplastischen Harz in einer Konzentration aufgelöst wird, die wenigstens das 20fache einer Konzentration in der endgültigen primären Harzzusammensetzung ist.

3. Verfahren nach Anspruch 2, bei dem das Mikrobiocid in dem wasserlöslichen Harz in einer Konzentration aufgelöst wird, die wenigstens das 100fache seiner Konzentration in der endgültigen primären Harzzusammensetzung ist.

4. Verfahren nach einem der vorausgehenden Ansprüche, bei dem das Mikrobiocid ein wasserunlösliches Mikrobiocid ist.

5. Verfahren nach einem der vorausgehenden Ansprüche, bei dem das Mikrobiocid ein Phenoxazin oder ein Phenarsazin ist.

6. Verfahren nach Anspruch 5, bei dem das Mikrobiocid 10,10'-Oxybisphenoxarsin ist.

7. Verfahren nach einem der vorausgehenden Ansprüche, bei dem das wasserlösliche Polymerharz ein thermoplastisches Harz auf Polyvinylalkoholbasis ist.

8. Verfahren nach einem der vorausgehenden Ansprüche, bei dem das Mikrobiocid in dem Konzentrat in einer Konzentration von wenigstens 2 Gew.%, vorzugsweise wenigstens 5 Gew.% vorliegt.

9. Polymere Harzzusammensetzung, erhalten durch ein Verfahren nach einem der vorausgehenden Ansprüche, mit einem primären thermoplastischen Harz oder Gemisch solcher Harze, wenigstens 0,03 Gew.% eines Mikrobiocids, um die Zusammensetzung vor oberflächlichem Mikrobenwachstum zu schützen, und einem thermoplastischen Polymerharz, welches in Wasser bis zu wenigstens 1 g/100 ml Wasser bei 25 °C löslich ist.

10. Zusammensetzung nach Anspruch 9, worin das thermoplastische Harz oder die thermoplastischen Harze halogeniertes Vinylharz sind.

11. Zusammensetzung nach Anspruch 10, worin das primäre thermoplastische Harz ein Polyvinylchlorid ist.

12. Zusammensetzung nach einem der Ansprüche 9 bis 11, worin das wasserlösliche thermoplastische Harz ein thermoplastisches Harz auf Polyvinylalkoholbasis ist.

13. Zusammensetzung nach einem der Ansprüche 9 bis 12, worin das Mikrobiocid ein Phenarsazin oder ein Phenoxarsin ist.

14. Zusammensetzung nach Anspruche 13, worin das primäre thermoplastische Harz unter Acrylnitril-Butadien-Styrolpolymer, Polyethylenterephthalat, Polystyrol, Styrol-Butadienkautschuk, Polyvinylchlorid, Polyvinylidenchlorid und Ethylenvinylacetatcopolymer ausgewählt ist.

15. Zusammensetzung nach einem der Ansprüche 9 bis 14, worin die Mikrobiocidkonzentration 0,03 bis 0,5 Gew.% beträgt.

## Patentansprüche (Patentansprüche für folgende(n) Vertragsstaat(en): ES)

1. Verfahren zum Dispergieren eines festen Mikrobiocids in einer starren thermoplastischen Harzzusammensetzung, wobei das Mikrobiocid in einem Träger unter Bildung eines Konzentrates aufgelöst wird und das Konzentrat in der Harzzusammensetzung dispergiert wird, **dadurch gekennzeichnet**, daß das Konzentrat eine feste Lösung oder Suspension von Mikrokristallen des Mikrobiocids in einem thermoplastischen Polymerharz ist, welches in Wasser bis zu wenigstens 1 g/100 ml Wasser bei 25 °C löslich ist.

2. Verfahren nach Anspruch 1, bei dem das Konzentrat in einem starren primären thermoplastischen Harz dispergiert wird und das Mikrobiocid in diesem wasserlöslichen thermoplastischen Harz in einer Konzentration aufgelöst wird, die wenigstens das 20fache einer Konzentration in der endgültigen primären Harzzusammensetzung ist.

3. Verfahren nach Anspruch 2, bei dem das Mikrobiocid in dem wasserlöslichen Harz in einer Konzentration aufgelöst wird, die wenigstens das 100fache seiner Konzentration in der endgültigen primären Harzzusammensetzung ist.

4. Verfahren nach einem der vorausgehenden Ansprüche, bei dem das Mikrobiocid ein wasserunlösliches Mikrobiocid ist.

5. Verfahren nach einem der vorausgehenden Ansprüche, bei dem das Mikrobiocid ein Phenoxazin oder ein Phenarsazin ist.

6. Verfahren nach Anspruch 5, bei dem das Mikrobiocid 10,10'-Oxybisphenoxarsin ist.

7. Verfahren nach einem der vorausgehenden Ansprüche, bei dem das wasserlösliche Polymerharz ein thermoplastisches Harz auf Polyvinylalkoholbasis ist.

8. Verfahren nach einem der vorausgehenden Ansprüche, bei dem das Mikrobiocid in dem Konzentrat in einer Konzentration von wenigstens 2 Gew.%, vorzugsweise wenigstens 5 Gew.% vorliegt.

9. Verfahren nach einem der vorausgehenden Ansprüche, worin das primäre thermoplastische Harz ein halogeniertes Vinylharz ist.

10. Verfahren nach einem der Ansprüche 1 bis 8, bei dem das primäre thermoplastische Harz unter Acrylnitril-Butadien-Styrolpolymer, Polyethylenterephthalat, Polystyrol, Styrol-Butadienkautschuk, Polyvinylchlorid, Polyvinylidenchlorid und Ethylenvinylacetatcopolymer ausgewählt ist.

11. Verfahren nach einem der vorausgehenden Ansprüche, bei dem die Mikrobiocidkonzentration in der letztendlichen Harzzusammensetzung 0,03 bis 0,5 Gew.% beträgt.

## Revendications (Revendications pour l'(les) Etat(s) contractant(s) suivant(s): AT, BE, CH, DE, DK, FR, GB, GR, IT, LI, LU, NL, SE)

1. Procédé de dispersion d'un microbicide solide dans une composition de résine thermoplastique rigide dans lequel le microbicide est dissous dans un support pour former un concentré et le concentré est dispersé dans ladite composition de résine, caractérisé en ce que ledit concentré est une solution ou suspension solide de microcristaux du microbicide dans une résine polymérique thermoplastique qui est soluble dans l'eau à raison d'au moins 1 g pour 100 ml d'eau à 25°C.

2. Procédé selon la revendication 1, dans lequel le concentré est dispersé dans une résine thermoplastique primaire rigide et le microbicide est dissous dans ladite résine thermoplastique soluble dans l'eau à une concentration qui est au moins 20 fois sa concentration dans la composition de résine primaire finale.

3. Procédé selon la revendication 2, dans lequel le microbicide est dissous dans ladite résine soluble dans l'eau à une concentration qui est au moins 100 fois sa concentration dans la composition de résine primaire finale.

4. Procédé selon l'une quelconque des revendications précédentes, dans lequel ledit microbicide est un microbicide insoluble dans l'eau.

5. Procédé selon l'une quelconque des revendications précédentes, dans lequel ledit microbicide est une phénoxarsine ou une phénarsazine.

6. Procédé selon la revendication 5, dans lequel ledit microbicide est la 10,10'-oxybisphénoxarsine.

7. Procédé selon l'une quelconque des revendications précédentes, dans lequel ladite résine polymérique soluble dans l'eau est une résine thermoplastique à base de poly(alcool vinylique).

8. Procédé selon l'une quelconque des revendications précédentes, dans lequel ledit microbicide est présent dans le concentré à une concentration d'au moins 2 % en masse, de préférence d'au moins 5 % en masse.

9. Composition de résine polymérique qui peut être obtenue par un procédé selon l'une quelconque des revendications précédentes comprenant une résine thermoplastique primaire ou un mélange de telles résines, au moins 0,03 % en masse d'un microbicide pour protéger ladite composition contre la croissance microbienne de surface et une résine polymérique thermoplastique qui est soluble dans l'eau à raison d'au moins 1 g pour 100 ml d'eau à 25°C.

10. Composition selon la revendication 9, dans laquelle ladite ou lesdites résines thermoplastiques primaires est ou sont une ou des résines vinyliques halogénées.

11. Composition selon la revendication 10, dans laquelle ladite résine thermoplastique primaire est un poly(chlorure de vinyle).

12. Composition selon l'une quelconque des revendications 9 à 11, dans laquelle ladite résine thermoplastique soluble dans l'eau est une résine thermoplastique à base de poly(alcool vinylique).

13. Composition selon l'une quelconque des revendications 9 à 12, dans laquelle le microbicide est une phénarsazine ou une phénoxarsine.

14. Composition selon la revendication 13, dans laquelle ladite résine thermoplastique primaire est choisie parmi un polymère acrylonitrile-butadiène-styrène, le polyéthylènetéréphtalate, le polystyrène, un caoutchouc styrène-butadiène, le poly(chlorure de vinyle), le poly(chlorure de vinylidene) et un copolymère éthylène-acétate de vinyle.

15. Composition selon l'une quelconque des revendications 9 à 14, dans laquelle la concentration en microbicide est de 0,03 à 0,5 % en masse.

## Revendications (Revendications pour l'(les) Etat(s) contractant(s) suivant(s): ES)

1. Procédé de dispersion d'un microbicide solide dans une composition de résine thermoplastique rigide dans lequel le microbicide est dissous dans un support pour former un concentré et le concentré est dispersé dans ladite composition de résine, caractérisé en ce que ledit concentré est une solution ou suspension solide de microcristaux du microbicide dans une résine polymérique thermoplastique qui est soluble dans l'eau à raison d'au moins 1 g pour 100 ml d'eau à 25°C.

2. Procédé selon la revendication 1, dans lequel le concentré est dispersé dans une résine thermoplastique primaire rigide et le microbicide est dissous dans ladite résine thermoplastique soluble dans l'eau à une concentration qui est au moins 20 fois sa concentration dans la composition de résine primaire finale.

3. Procédé selon la revendication 2, dans lequel le microbicide est dissous dans ladite résine soluble dans l'eau à une concentration qui est au moins 100 fois sa concentration dans la composition de résine primaire finale.

4. Procédé selon l'une quelconque des revendications précédentes, dans lequel ledit microbicide est un microbicide insoluble dans l'eau.

5. Procédé selon l'une quelconque des revendications précédentes, dans lequel ledit microbicide est une phénoxarsine ou une phénarsazine.

6. Procédé selon la revendication 5, dans lequel ledit microbicide est la 10,10'-oxybisphénoxarsine.

7. Procédé selon l'une quelconque des revendications précédentes, dans lequel ladite résine polymérique soluble dans l'eau est une résine thermoplastique à base de poly(alcool vinylique).

8. Procédé selon l'une quelconque des revendications précédentes, dans lequel ledit microbicide est présent dans le concentré à une concentration d'au moins 2 % en masse, de préférence d'au moins 5 % en masse.

9. Procédé selon l'une quelconque des revendications précédentes, dans lequel ladite résine thermoplastique primaire est une résine vinylique halogénée.

10. Procédé selon l'une quelconque des revendications 1 à 8, dans lequel ladite résine thermoplastique primaire est choisie parmi un polymère acrylonitrile-butadiène-styrène, le polyéthylènetéréphtalate, le polystyrène, un caoutchouc styrène-butadiène, le poly(chlorure de vinyle), le poly(chlorure de vinylidene) et un copolymère éthylène-acétate de vinyle.

11. Procédé selon l'une quelconque des revendications précédentes, dans lequel la concentration du microbicide dans la composition de résine finale est de 0,03 à 0,5 % en masse.
